# EUROPÄISCHE PATENTSCHRIFT

(11) **EP 1 706 382 B1**
(45) Veröffentlichungstag und Bekanntmachung des Hinweises auf die Patenterteilung: **12.03.2008**
(21) Anmeldenummer: 04804128.9
(22) Anmeldetag: 21.12.2004
(51) Int. Cl.: C07D 213/61

(54) **VERFAHREN ZUR STABILISIERUNG VON 4-HALOGENPYRIDINEN MIT SEKUNDÄREN UND/ODER TERTIÄREN AMINEN**
METHOD FOR STABILIZING 4-HALOGENPYRIDINES WITH SECONDARY AND/OR TERTIARY AMINES
PROCEDE POUR STABILISER DES 4-HALOGENOPYRIDINES AVEC DES AMINES SECONDAIRES ET/OU TERTIAIRES

(30) Priorität: 13.01.2004 DE 102004001950
(43) Veröffentlichungstag der Anmeldung: 04.10.2006
(73) Patentinhaber: Sanofi-Aventis Deutschland GmbH, 65929 Frankfurt am Main (DE)
(72) Erfinder: DUBBERKE, Silke, 65439 Flörsheim (DE); MANERO, Javier, 65832 Liederbach (DE)
(86) Internationale Anmeldenummer: PCT/EP2004/014530
(87) Internationale Veröffentlichungsnummer: WO 2005/068428

(56) Entgegenhaltungen:
- US-A- 3 703 521
- LYLE ET AL: "Synthesis of 4-fluorolutidines and 4-fluoropyridine" JOURNAL OF HETEROCYCLIC CHEMISTRY, Bd. 9, Nr. 3, 1972, Seiten 745-746, XP002321456
- URGAONKAR ET AL: "Application of a New Bicyclic Triaminophosphine Ligand in Pd-Catalyzed Buchwald-Hartwig Amination Reactions of Aryl Chlorides, Bromides, and Iodides" JOURNAL OF ORGANIC CHEMISTRY, Bd. 68, Nr. 22, 2003, Seiten 8416-8423, XP002321457
- CHAHMA ET AL: "Use of pyrrole anions as nucleophiles in electrochemically induced SRN1 reactions" TETRAHEDRON LETTERS, Bd. 32, Nr. 43, 1991, Seiten 6121-6124, XP002321458

## Beschreibung

4-Halogenpyridine sind bei Lagerung bei Raumtemperatur instabile Verbindungen, die synthetisch über an sich bekannte Methoden, beispielsweise durch Chlorierung des entsprechenden 4-Hydroxypyridins mit PCl₅ oder POCl₃ zugänglich sind (Wibaut & Broekmann, Recueil des Travaux Clinique des Pays-Bas et de la Belgique 7659, 78, 593-603). Verunreinigungen aus dem Herstellungsverfahren, insbesondere Reste von Säure, führen zur Zersetzung der 4-Halogenpyridine und zur Bildung von oligomeren Produkten. Im Fall von 4-Chlorpyridin entsteht unter anderem das Salz des Dimers N-(4'-pyridyl)-4-chlorpyridiniumchlorid (Wibaut & Broeckmann, Recueil des Travaux Clinique des Pays-Bas et de la Belgique 1961, 80, 309-312).

Den Hertog et al. (Wibaut & Brockmann, Recueil des Travaux Clinique des Pays-Bas et de la Belgique 1951, 70, 105-111) beschreiben, dass 4-Nitropyridin durch den Zusatz von 10%iger wässriger Natriumbicarbonat-Lösung stabilisiert werden kann, wobei nach 12 Stunden bei 60°C noch 90% 4-Nitropyridin unverändert vorlagen.

Laut Wibaut & Brockmann (Recueil des Travaux Clinique des Pays-Bas et de la Belgique 1961, 80, 309-312) beobachtet man in Gemischen von 4-Chlorpyridin mit primären Aminen NH₂R (R=CH₃, CH(CH₃)₂, C₁₂H₂₅) oder sekundären Aminen NHR'₂ (R'=CH₃, n-C₄H₉) die Bildung der entsprechenden (4-NHR)-bzw. (4-NR'₂)-Pyridine, während aliphatische tertiäre oder gemischt aliphatisch-aromatische tertiäre Amine (NEt₃, Diethylanilin) mit 4-Chlorpyridin nicht reagierten. 4-Chlorpyridin bildet mit dem aromatischen tertiären Amin Pyridin ein N-(4'-pyridyl)-pyridinium-Salz. 4-Chlorpyridin ist zur Zeit nur als Hydrochlorid käuflich, wobei die veränderten elektronischen Verhältnisse im Pyridiniumsalz für die Stabilisierung sorgen.

Die Erfindung betrifft ein Verfahren zur Stabilisierung von 4-Halogenpyridinen der Formel (I) 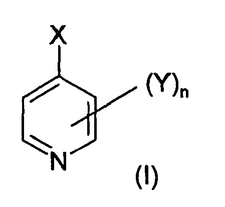 wobei
- X: Fluor, Chlor, Brom oder Iod,
- Y: Wasserstoff, Fluor, Chlor, Brom, Iod, Nitro, NH₂, (C₁-C₄)-Alkyl, (C₁-C₄)-Alkanoylamin, Phenyl-(C₁-C₄)-alkanoylamin, Phenylcarbonylamin, (C₁-C₄)-Alkylamin, di-(C₁-C₄)-Alkylamin, Phenyl-(C₁-C₄)-alkylamin, und
- n: 1 oder 2
bedeutet,
dadurch gekennzeichnet, dass eine oder mehrere Verbindungen der Formel (I) mit einem oder mehreren sekundären und/oder tertiären aliphatischen oder gemischt aliphatisch-aromatischen Aminen versetzt werden.

In dem Verfahren ist unabhängig voneinander
- X: bevorzugt Cl,
- Y: bevorzugt Wasserstoff oder Fluor, und
- n: bevorzugt 1.

Bevorzugt wird in dem Verfahren jeweils eine Verbindung der Formel (I) und ein Amin eingesetzt.
Besonders bevorzugt ist ein Verfahren, wobei die Verbindung der Formel (I) durch die Formel (II) oder (III) beschrieben wird: 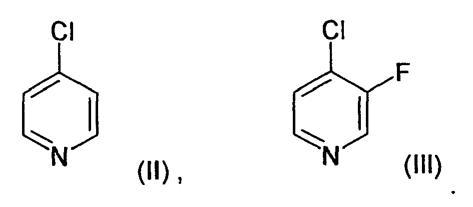 In dem oben genannten Verfahren zur Stabilisierung bzw. in dem oben genannten Gemisch können alle dem Fachmann bekannte sekundären Amine, oder bevorzugt aliphatische oder gemischt aliphatisch-aromatische tertiären Amine verwendet werden.

Ein sekundäres Amin ist beispielsweise Diisopropylamin, Morpholin, Pyrrolidin, Piperidin, N,N-Dicyclohexylamin, vorzugsweise Diisopropylamin.

Ein tertiäres aliphatisches Amin ist beispielsweise N-Methylmorpholin, Ethyldiisopropylamin, N,N,N',N'-Tetramethylethylendiamin (TMEDA), 1,5-Diazabicyclo[4.3.0]-5-nonen (DBN); 1,8-Diazabicyclo[5.4.0]-7-undecen (DBU), Dicyclohexylethylamin, Urotropin, vorzugsweise TMEDA, N-Methylmorpholin und Ethyldiisopropylamin.

Ein tertiäres gemischt aliphatisch-aromatisches Amin ist beispielsweise Dimethylaminopyridin (DMAP), N,N-Dimethylanilin, vorzugsweise DMAP.

Besonders bevorzugt wird in dem erfindungsgemäßen Verfahren Tetramethylethylendiamin (TMEDA), N-Methylmorpholin und Ethyldiisopropylamin verwendet.

Die Verbindungen der Formel (I) sind von Nutzen als Ausgangsmaterial in der Synthese von pharmazeutischen Produkten. Beispielsweise ist die Verwendung der Verbindung (II) und (III) in der Herstellung von Verbindungen, die in den europäischen Patentanmeldungen EP 287 982 beschrieben sind und zur Behandlung von Schmerz und Depression angewandt werden können. Dabei ist es von Vorteil, wenn das 4-Halogenpyridin-Ausgangsmaterial insbesondere erstens lange lagerfähig ist, zweitens keine Quelle von Verunreinigungen (z. B. von Zersetzungsprodukten) ist, die das pharmazeutische Produkt verunreinigen könnten, und drittens sich nicht als exotherm zersetzliches Material als Sicherheitsrisiko erweist.

4-Chlor-3-fluorpyridin kann beispielsweise in der Herstellung der Verbindung N-(3-Fluor-4-pyridinyl)-N-propyl-3-methyl-1 H-indol-1-amin verwendet werden, die in der Behandlung von Nervenschädigungen Verwendung findet. Der Chlorsubstituent wird dabei unter Zusatz einer Base durch ein Amin ersetzt.

In dem erfindungsgemäßen Verfahren zur Stabilisierung von Halogenpyridinen bzw. dem Gemisch ist ein Gehalt von bis zu 10%, vorzugsweise 5% Amin bevorzugt.

Die begrenzte Stabilität und das erfindungsgemäße Verfahren zur Steigerung der Stabilität wird im folgenden beispielhaft an den Verbindungen 4-Chlorpyridin und 4-Chlor-3-fluorpyridin (4-CIFPy) erläutert. Die Zersetzungsprodukte von 4-CIFPy wurden genauer untersucht.

Zur Aufklärung der Zersetzungsprodukte wurde der bei Lagerung ausgefallene Feststoff abgesaugt und anschließend chromatographisch getrennt. Hierzu wurde der Feststoff in Triethylamin gelöst, mit dem Laufmittel Ethylacetat/Ethanol 4:1 über Kieselgel getrennt. Hierbei fielen zwei Fraktionen an. Fraktion 1 mit R_{f} = 0,83 und Fraktion 2 mit R_{f} = 0,53 im genannten Laufmittelsystem. Die beiden Fraktionen konnten als die folgenden Dimerisierungs-/Isomerisierungsprodukte aufgeklärt werden:

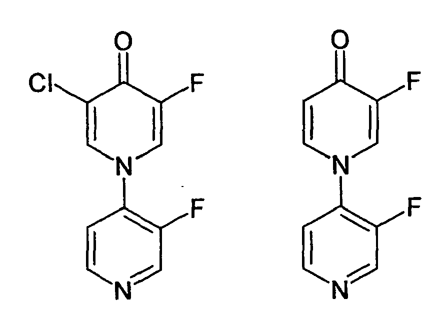

Zwei Chargen von 4-CIFPy mit unterschiedlichem Gehalt an TMEDA wurden hinsichtlich ihrer Lagerstabilität bei 20-25°C untersucht.

**Tabelle 1:**

| Charge | Gehalt TMEDA | Zeitpunkt | Gehalt 4-CIFRy | Reinheit (GC) |
|---|---|---|---|---|
| 1 | 2,15 % | Beginn d. Lagerung | 98,8% | 97,8% |
| | | +5 Monate | 98,2 % | 97,9% |
| 2 | 0,47 % | Beginn d. Lagerung | 98,9 % | 99,4 % |
| | | +3 Monate | 100,9% | 99,5% |
| | | +9 Monate | 99,4% | 99,3% |

Im Bereich der Messungenauigkeit veränderte sich der Gehalt an 4-Chlor-3-fluorpyridin während des beobachteten Zeitraums nicht, das aliphatische tertiäre Amin TMEDA ist somit in der Lage, 4-halogen-substituierte Pyridine zu stabilisieren.

Um den beobachteten Effekt für weitere tertiäre und sekundäre Amine zu verifizieren, wurden die Amine Triethylamin, N-Methylmorpholin, Ethyldiisopropylamin und Diisopropylamin in unterschiedlichen Zumischungen (A: 10 µl auf 5 ml; B: 10 µl auf 1 ml) jeweils einer Probe von 4-CIFPy zugesetzt, und der Gehalt an Zersetzungsprodukten nach Lagerung bei Raumtemperatur mittels Gaschromatographie (GC) bestimmt (Blindwert: 0,097%):

**Tabelle 2: Stabilisierung von 4-CIFPy durch Amine**

| | | Zeitraum der Lagerung [Tage] | | | | | | |
|---|---|---|---|---|---|---|---|---|
| | | 3 | 10 | 17 | 24 | 31 | 59 | 80 |
| Amin | Menge | Gehalt Zersetzungsprodukte [%] | | | | | | |
| NEt₃ | A | 0,11 | 0,11 | 0,12 | 0,13 | 0,13 | 0,09 | 0,11 |
| | B | 0,11 | 0,12 | 0,12 | 0,13 | 0,13 | 0,09 | 0,11 |
| N-Methyl-morpholin | A | 0,11 | 0,12 | 0,12 | 0,12 | 0,13 | 0,07 | 0,10 |
| | B | 0,11 | 0,12 | 0,12 | 0,12 | 0,13 | 0,15 | 0,10 |
| Et(i-Pr)₂N | A | 0,11 | 0,12 | 0,12 | 0,12 | 0,13 | 0,16 | 0,10 |
| | B | 0,11 | 0,12 | 0,13 | 0,13 | 0,13 | 0,16 | 0,11 |
| (i-Pr)₂NH | A | 0,11 | 0,12 | 0,11 | 0,12 | 0,13 | 0,16 | 0,10 |
| | B | 0,11 | 0,12 | 0,11 | 0,13 | 0,13 | 0,17 | 0,11 |
| TMEDA | A | 0,11 | 0,11 | 0,11 | 0,12 | 0,13 | 0,16 | 0,10 |
| | B | 0,11 | 0,12 | 0,12 | 0,16 | 0,2 | 0,22 | 0,12 |

**Tabelle 3: Lagerung von 4-CIFPy (Kontrollversuch)**

| | Zeitraum der Lagerung [Tage] | | | | | | |
|---|---|---|---|---|---|---|---|
| Lagerung ohne Amin [°C] | 3 | 10 | 17 | 24 | 31 | 59 | 80 |
| 20-25°C | 0,13 | 0,21 | 0,34 | 0,71 | 1,5 | 0,72 | 0,41 |
| 0-5°C | 0,11 | 0,14 | 0,13 | 0,17 | 0,22 | 0,38 | 0,17 |

Bei der Lagerung bei 20-25°C im Kontrollversuch konnte bei Tag 31 Feststoff festgestellt werden, ebenfalls wurde Feststoff in der Kühlschrankprobe (0-5°C) ab Tag 59 beobachtet werden. Sobald Feststoff in der Probe vorhanden war, war es nicht mehr möglich eine homogene Probe zu ziehen, so dass die Nebenkomponente im GC unterrepräsentiert ist. Bei den folgenden Messungen wurden somit niedrigere Werte als zuvor gefunden.

Die Schwankungen bei den vorherigen Messungen liegen im Fehlerbereich der Integration des Gaschromatographen. Bereits geringe Mengen Amin reichen aus, um 4-Chlor-3-fluorpyridin zu stabilisieren. Als Beispiel seien die zwei unterschiedlichen Konzentrationen von TMEDA genannt, im verdünnten Fall A werden nur 0,13 % TMEDA detektiert. Die konzentrierte Lösung B zeigt im Vergleich 0,61 % TMEDA an.

Aus den oben beschriebenen Versuchen ergibt sich, dass der Zusatz eines sekundären Amins und/oder eines tertiären aliphatischen oder gemischt aliphatisch-aromatischen Amins im Konzentrationsbereich von insgesamt 0,05-5,0%, vorzugsweise 0,1-2,5%, ein 4-Halogenpyridin-Derivat der Formel (I), vorzugsweise eine Verbindung der Formel (II) und (III), stabilisiert.

### Beispiel 1: Charakterisierung von 4-Chlor-3-fluorpyridin

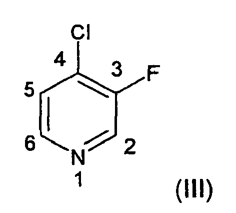

| ¹H NMR: | | | |
|---|---|---|---|
| H2 | 8.5 ppm | J(H2-H6)= 0Hz, J(H2-H5)= 0Hz | 3JFH=1 Hz |
| H5 | 7.4 ppm | J(H5-H6)= 5.3Hz, J(H5-H2)= OHz | 4JFH=0Hz |
| H6 | 8.3 ppm | J(H6-H2)= 0Hz, J(H6-H5)= 5.3Hz | SJFH=6.0Hz |

| 13C NMR: | | |
|---|---|---|
| C2 | 140 ppm | 2JFC=23Hz |
| C3 | 156 ppm | 1JFC=259Hz |
| C4 | 131 ppm | 2JFC=15Hz |
| C5 | 126 ppm | 3JFC=2.3Hz |
| C6 | 146 ppm | 4JFC=5.7Hz |

| ¹⁹F NMR: | |
|---|---|
| F3 | 131 ppm |

### Beispiel 2: Charakterisierung der Dimerisierungs-/Isomerisierungsprodukte durch NMR-chemische Verschiebungen

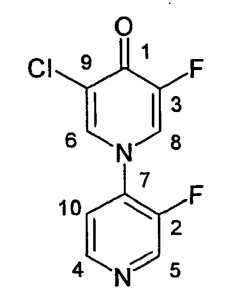

| ¹H NMR | | ¹³C NMR | |
|---|---|---|---|
| H4 | 8.65 ppm, d | C1 | 164.06 ppm |
| H5 | 8.8 ppm, d | C2 | 151.76 ppm |
| H6 | 8.61 ppm, t | C3 | 151.58 ppm |
| H8 | 8.62 ppm, t | C4 | 147.29 ppm |
| H10 | 7.8 ppm, dd | C5 | 139.77 ppm |
| | | C6 | 137.8 ppm |
| | | C7 | 135.45 ppm |
| | | C8 | 127.51 ppm |
| | | C9 | 123.94 ppm |
| | | C10 | 121.06 ppm |

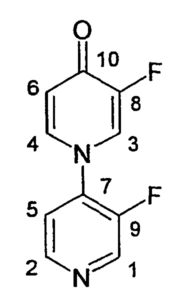

| ¹H NMR | | ¹³C NMR | |
|---|---|---|---|
| H1 | 8.86 ppm, d | C1 | 139.56 ppm |
| H2 | 8.63 ppm, d | C2 | 147.09 ppm |
| H3 | 8.48 ppm, ddd | C3 | 126.97 ppm |
| H4 | 8.02 ppm, ddd | C4 | 139.13 ppm |
| H5 | 7.81 ppm, dd | C5 | 120.4 ppm |
| H6 | 6.53 ppm, dd | C6 | 118.3 ppm |
| | | C7 | 135.9 ppm |
| | | C8 | 153.38 ppm |
| | | C9 | 150.8 ppm |
| | | C10 | 168.95 ppm |

## Patentansprüche

1. Verfahren zur Stabilisierung von 4-Halogenpyridinen der Formel (I) 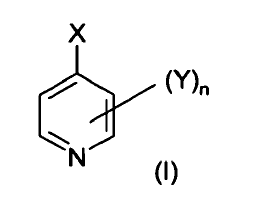 wobei
X Fluor, Chlor, Brom oder Iod,
Y Wasserstoff, Fluor, Chlor, Brom, Iod, Nitro, NH₂, (C₁-C₄)-Alkyl, (C₁-C₄)-Alkanoylamin, Phenyl-(C₁-C₄)-alkanoylamin, Phenylcarbonylamin, (C₁-C₄)-Alkylamin, di-(C₁-C₄)-Alkylamin, Phenyl-(C₁-C₄)-alkylamin, und
n 1 oder 2
bedeutet,
**dadurch gekennzeichnet, dass** eine oder mehrere Verbindungen der Formel (I) mit einem oder mehreren sekundären und/oder tertiären aliphatischen oder gemischt aliphatisch-aromatischen Aminen versetzt werden.

2. Verfahren gemäß Anspruch 1, wobei X gleich Cl ist.

3. Verfahren gemäß einem der Ansprüche 1 oder 2, wobei Y gleich Wasserstoff oder Fluor ist.

4. Verfahren gemäß einem der Ansprüche 1 bis 3, wobei n gleich 1 ist.

5. Verfahren gemäß einem der Ansprüche 1 bis 4, wobei die Verbindung der Formel (1) eine Verbindung der Formel (II) oder der Formel (III) ist

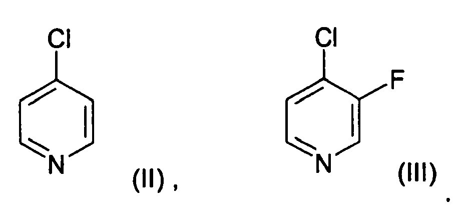

6. Verfahren gemäß einem der Ansprüche 1 bis 5, wobei jeweils eine Verbindung der Formel (I) und ein Amin eingesetzt werden.

7. Verfahren gemäß einem der Ansprüche 1 bis 6, wobei der Gehalt an sekundärem Amin und/oder an tertiärem aliphatischem oder gemischt aliphatisch-aromatischem Amin 0,05-5,0% insgesamt beträgt.

8. Verfahren gemäß einem der Ansprüche 1 bis 7, wobei das sekundäre Amin Diisopropylamin, Morpholin, Pyrrolidin, Piperidin oder N,N-Dicyclohexylamin ist.

9. Verfahren gemäß einem der Ansprüche 1 bis 7, wobei das tertiäre aliphatische Amin N-Methylmorpholin, Ethyldiisopropylamin. N,N,N',N'-Tetramethylethylendiamin (TMEDA), 1,5-Diazabicyclo[4.3.0]-5-nonen (DBN); 1,8-Diazabicyclo[5.4.0]-7-undecen (DBU), Dicyclohexylethylamin oder Urotropin ist.

10. Verfahren gemäß einem der Ansprüche 1 bis 7, wobei das tertiäre gemischt aliphatisch-aromatisches Amin Dimethylaminopyridin (DMAP) oder N,N-Dimethylanilin ist.

## Claims

1. A process for stabilizing 4-halopyridines of the formula (I) 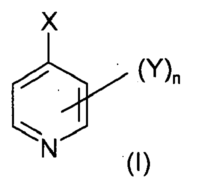 where
X is fluorine, chlorine, bromine or iodine,
Y is hydrogen, fluorine, chlorine, bromine, iodine, nitro, NH₂, (C₁-C₄)-alkyl, (C₁-C₄)-alkanoylamine, phenyl-(C₁-C₄)-alkanoylamine, phenylcarbonylamine, (C₁-C₄)-alkylamine, di-(C₁-C₄)-alkylamine, phenyl-(C₁-C₄)-alkylamine, and
n is 1 or 2,
which comprises admixing one or more compounds of the formula (I) with one or more secondary and/or tertiary aliphatic or mixed aliphatic-aromatic amines.

2. The process as claimed in claim 1, wherein X is Cl.

3. The process as claimed in one of claims 1 and 2, wherein Y is hydrogen or fluorine.

4. The process as claimed in one of claims 1 to 3, wherein n is 1.

5. The process as claimed in one of claims 1 to 4, wherein the compound of the formula (I) is a compound of the formula (II) or of the formula (III)

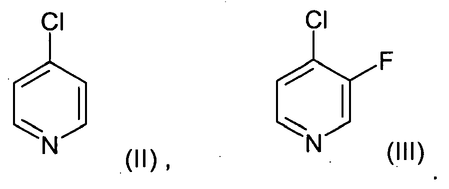

6. The process as claimed in one of claims 1 to 5, wherein in each case one compound of the formula (I) and one amine are used.

7. The process as claimed in one of claims 1 to 6, wherein the content of secondary amine and/or of tertiary aliphatic or mixed aliphatic-aromatic amine is 0.05-5.0% in total.

8. The process as claimed in one of claims 1 to 7, wherein the secondary amine is diisopropylamine, morpholine, pyrrolidine, piperidine or N,N-dicyclohexylamine.

9. The process as claimed in one of claims 1 to 7, wherein the tertiary aliphatic amine is N-methylmorpholine, ethyldiisopropylamine, N,N,N',N'-tetramethylethylenediamine (TMEDA), 1,5-diazabicyclo[4.3.0]-5-nonene (DBN); 1,8-diazabicyclo[5.4.0]-7-undecene (DBU), dicyclohexylethylamine or urotropine.

10. The process as claimed in one of claims 1 to 7, wherein the tertiary mixed aliphatic-aromatic amine is dimethylaminopyridine (DMAP) or N,N-dimethylaniline.

## Revendications

1. Procédé pour la stabilisation de 4-halogénopyridines de formule (I) 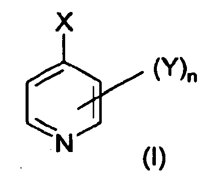 où
X signifie fluor, chlore, brome ou iode,
Y signifie hydrogène, fluor, chlore, brome, iode, nitro, NH₂, (C₁-C₄) -alkyle, (C₁-C₄)-alcanoylamine, phényl-(C₁-C₄)-alcanoylamine, phénylcarbonylamine, (C₁-C₄) -alkylamine, di- (C₁-C₄) -alkylamine, phényl-(C₁-C₄) -alkylamine, et
n signifie 1 ou 2,
**caractérisé en ce qu'**un ou plusieurs composés de formule (I) sont mélangés avec une ou plusieurs amines secondaires aliphatiques ou aliphatiques-aromatiques mixtes et/ou tertiaires.

2. Procédé selon la revendication 1, où X représente Cl.

3. Procédé selon l'une quelconque des revendications 1 ou 2, où Y représente hydrogène ou fluor.

4. Procédé selon l'une quelconque des revendications 1 à 3, où n vaut 1.

5. Procédé selon l'une quelconque des revendications 1 à 4, où le composé de formule (I) est un composé de formule (II) ou de formule (III)

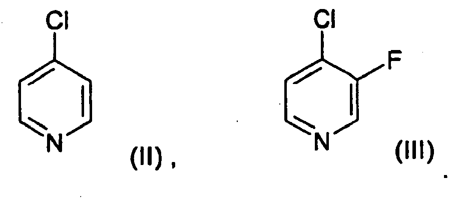

6. Procédé selon l'une quelconque des revendications 1 à 5, où on utilise à chaque fois un composé de formule (I) et une amine.

7. Procédé selon l'une quelconque des revendications 1 à 6, où la teneur en amine secondaire et/ou en amine aliphatique et/ou aliphatique-aromatique mixte tertiaire est au total de 0,05-5,0%.

8. Procédé selon l'une quelconque des revendications 1 à 7, où l'amine secondaire est la diisopropylamine, la morpholine, la pyrrolidine, la pipéridine ou la N,N-dicyclohexylamine.

9. Procédé selon l'une quelconque des revendications 1 à 7, où l'amine aliphatique tertiaire est la N-méthylmorpholine, l'éthyldiisopropylamine, la N,N,N',N'-tétraméthyléthylènediamine (TMEDA), le 1,5-diazabicyclo[4.3.0]-5-nonène (DBN), le 1,8-diazabicyclo[5.4.0]-7-undécène (DBU), la dicyclohexyléthylamine ou l'urotropine.

10. Procédé selon l'une quelconque des revendications 1 à 7, où l'amine aliphatique-aromatique mixte tertiaire est la diméthylaminopyridine (DMAP) ou la N,N-diméthylaniline.
